# EUROPEAN PATENT APPLICATION

(11) **EP 4 801 230 A1**
(43) Date of publication of application: **02.09.2026**
(21) Application number: 24882477.3
(22) Date of filing: 25.10.2024
(51) Int. Cl.: H10K 30/50, C07D 487/22, H10K 30/40, H10K 30/81, H10K 30/86, H10K 85/10, H10K 85/30, H10K 85/50, H10K 85/60

(54) **PHOTOELECTRIC CONVERSION ELEMENT AND PHOTOELECTRIC CONVERSION DEVICE**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 28.05.2024 JP 2024085998
(71) Applicant: Canon Kabushiki Kaisha, Tokyo 146-8501 (JP)
(72) Inventor: WATARIGUCHI, Kaname, Tokyo 146-8501 (JP); OHSAWA, Tatsuya, Tokyo 146-8501 (JP); MURANAKA, Norifumi, Tokyo 146-8501 (JP); KITAHARA, Michitaka, Tokyo 146-8501 (JP); NISHIDA, Tsutomu, Tokyo 146-8501 (JP)
(74) Representative: TBK
(86) International application number: PCT/JP2024/038045
(87) International publication number: WO 2025/089368

(57) **Abstract**

The photoelectric conversion element includes a layer containing a crystal having a perovskite structure. When the smaller one of the electrode area of the first electrode and the electrode area of the second electrode is represented by S [cm²], in a Nyquist plot based on the measurement results of an impedance, the maximum value R_{rec} [Ω] out of resistance values obtained by fitting an arc corresponding to the maximum value of a phase in a low frequency range with a parallel circuit of a resistance element and a constant phase element satisfies the following formula (E1): $1.0 \times {10}^{4} < {R}_{rec} \times S < 1.0 \times {10}^{7}$ and the maximum value R_{ct} [Ω] out of resistance values obtained by fitting an arc corresponding to the maximum value of the phase in a high frequency range with the parallel circuit of the resistance element and the constant phase element, and the R_{rec} [Ω] satisfy the following formula (E2). ${R}_{rec} / {R}_{ct} \geq 25$

## Description

### [Technical Field]

The present invention relates to a photoelectric conversion element and a photoelectric conversion apparatus.

### [Background Art]

In order to solve a problem of the depletion of fossil energy and a global environmental problem caused by the use of the fossil energy, investigations on a renewable and clean alternative energy source, such as solar energy, wind power, or water power, have been actively performed. In particular, an interest in a solar cell that directly changes sunlight into electrical energy has been increasing. The term "solar cell" as used herein means a battery that generates a current-voltage through utilization of a photovoltaic effect in which light energy is absorbed from sunlight to generate an electron and a hole.

Recently, an n-p diode-type silicon (Si) single crystal-based solar cell having a light energy conversion efficiency of more than 20% has been widely known, and has been actually used in solar power generation. However, the solar cell requires a high temperature treatment step and the price of a material itself is high, and hence there is a problem in that the cost per unit electric power is high. In addition, there is a problem with its supply property in terms of a silicon resource.

Meanwhile, a solar cell using an organic material (hereinafter also referred to as "organic solar cell") does not require the high temperature treatment step, and is a sheet-shaped substrate, which can be produced in a so-called roll to roll system. Accordingly, cost reduction is expected. However, further improvements in power generation efficiency and durability have been desired for practical use of the organic solar cell. In particular, the development of a perovskite solar cell including a crystal having a perovskite structure as a photoelectric conversion layer toward its practical use has been advanced because the cell is excellent in photoelectric conversion property.

In Patent Literature 1, there is a description of a technology including incorporating an organic semiconductor and a polymer compound having a glass transition temperature of 100°C or more into a hole-transporting layer to improve its peeling from an electrode.

In Patent Literature 2, there are descriptions of a configuration in which a layer formed of a quinacridone pigment is arranged between a perovskite layer and a hole-transporting layer, and the impedance measurement results of the configuration.

In Non Patent Literature 1, there is a description of a technology of a charge-transporting layer for an inverted layer that improves photoelectric conversion efficiency by doping PEDOT:PSS with nickel phthalocyanine having a substituent.

### [Citation List]

### [Patent Literature]

PTL 1: Japanese Patent Laid-Open No. 2018-170382
PTL 2: U.S. Patent Application Publication No. 2022/0285642

### [Non Patent Literature]

NPL 1: X.-F. Zhang, J. Mater. Chem. A, 2018, 6, 12515-12522

### [Summary of Invention]

### [Technical Problem]

According to investigations made by the inventors of the present invention, it has been found that there is room for improvement in durability in each of the photoelectric conversion elements described in Patent Literature 1, Patent Literature 2, and Non Patent Literature 1.

Accordingly, the present invention is directed to providing a photoelectric conversion element and a photoelectric conversion apparatus in each of which durability is improved.

### [Solution to Problem]

The above-mentioned provision is achieved by the present invention described below. That is, a photoelectric conversion element according to the present invention is
a photoelectric conversion element including: a first electrode; a second electrode; and a photoelectric conversion layer arranged between the first electrode and the second electrode, the photoelectric conversion layer containing a crystal having a perovskite structure,
wherein, when a smaller one of an electrode area of the first electrode and an electrode area of the second electrode is represented by S [cm²],
and an impedance is measured by applying an AC voltage with a DC voltage component V_{DC}=0 [V] and a root-mean-square of an AC voltage component Vᵣₘₛ=50 [mV] to the photoelectric conversion element while changing a frequency thereof from 1.0×10⁻² [Hz] to 1.0×10⁶ [Hz],
in a plot in which a horizontal axis represents a frequency [Hz] and a vertical axis represents a phase [deg] based on measurement results of the impedance,
a phase of the impedance has a maximum value in a low frequency range of 1.0×10⁻² to less than 1.0×10² [Hz],
and the phase of the impedance has a maximum value in a high frequency range of 1.0×10² to 1.0×10⁶ [Hz], and wherein, in a Nyquist plot in which a horizontal axis represents an impedance real part Z' [Ω] and a vertical axis represents an impedance imaginary part Z" [Ω] based on the measurement results of the impedance,
a maximum value R_{rec} [Ω] out of resistance values obtained by fitting an arc corresponding to the maximum value of the phase in the low frequency range with a parallel circuit of a resistance element and a constant phase element satisfies the following formula (E1): $1.0 \times {10}^{4} \leq {R}_{rec} \times S \leq 1.0 \times {10}^{7}$
and a maximum value R_{ct} [Ω] out of resistance values obtained by fitting an arc corresponding to the maximum value of the phase in the high frequency range with the parallel circuit of the resistance element and the constant phase element, and the R_{rec} [Ω] satisfy the following formula (E2). ${R}_{rec} / {R}_{ct} \geq 25$

### [Advantageous Effects of Invention]

According to the present invention, there can be provided the photoelectric conversion element that is improved in durability while having high initial photoelectric conversion efficiency.

### [Brief Description of Drawings]

[Fig. 1]

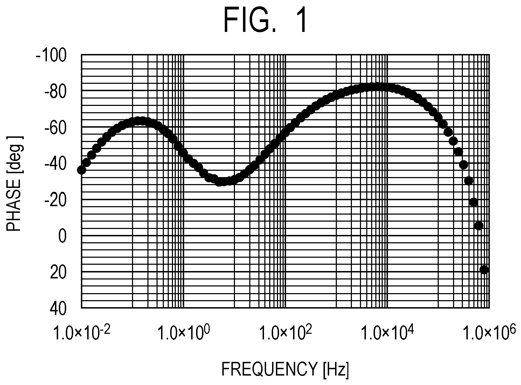

   Fig. 1 is a plot obtained in Example 4 of the present invention in which a horizontal axis of impedance data represents a frequency [Hz] and a vertical axis thereof represents a phase [deg].
[Fig. 2]

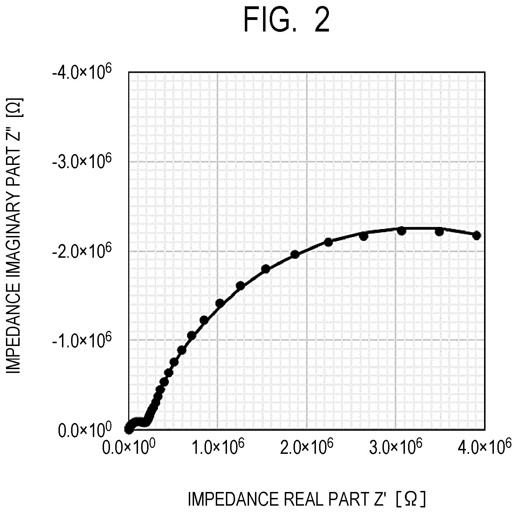

   Fig. 2 is a Nyquist plot of the impedance data and a fitting curve obtained in Example 4 of the present invention.
[Figs. 3]

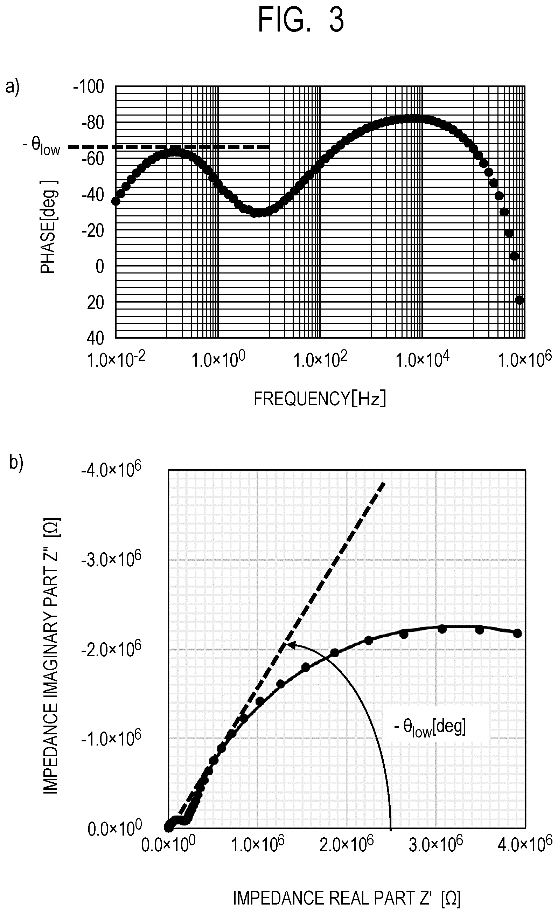

   Fig. 3(a) and Fig. 3(b) are each a diagram for describing the meaning of an arc corresponding to the maximum value of a phase in the present invention.
[Fig. 4]

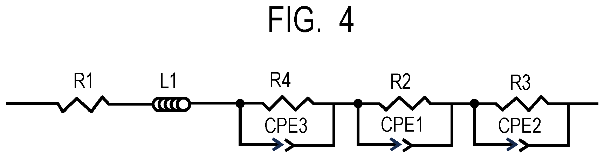

   Fig. 4 is an illustration of an equivalent circuit at the time of fitting in impedance analysis used in Examples of the present invention.
[Fig. 5]

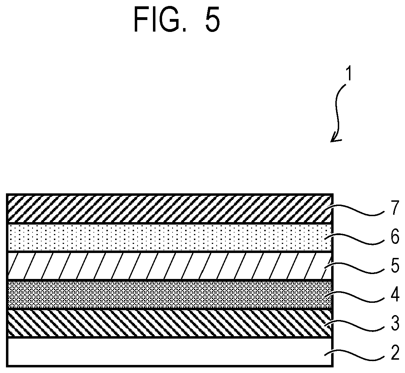

   Fig. 5 is a schematic sectional view in the thickness direction of a photoelectric conversion element according to a first embodiment of the present invention.
[Fig. 6]

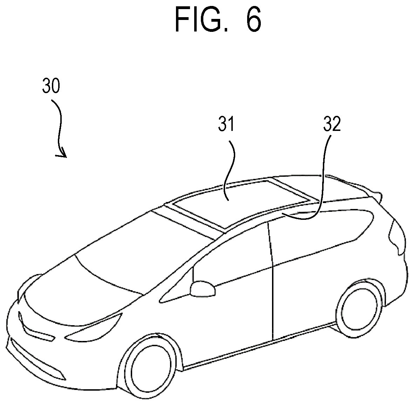

   Fig. 6 is a perspective view for schematically illustrating a moving body including the photoelectric conversion element according to one embodiment of the present invention.
[Fig. 7]

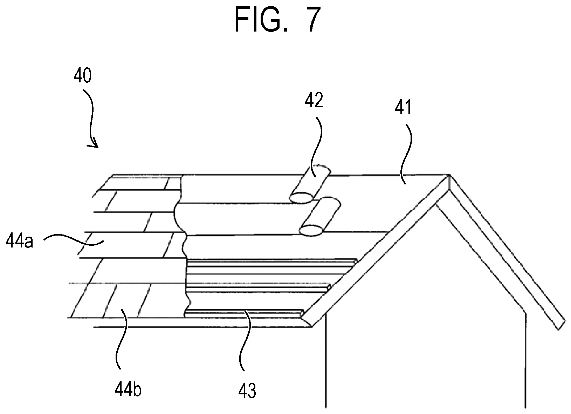

   Fig. 7 is a perspective view for schematically illustrating a building material including the photoelectric conversion element according to one embodiment of the present invention.

### [Description of Embodiments]

A photoelectric conversion element of the present invention is a photoelectric conversion element including: a first electrode; a second electrode; and a photoelectric conversion layer arranged between the first electrode and the second electrode, the layer containing a crystal having a perovskite structure, the element being characterized in that:
when the smaller one of the electrode area of the first electrode and the electrode area of the second electrode is represented by S [cm²],
and an impedance is measured by applying an AC voltage with a DC voltage component V_{DC}=0 [V] and a root-mean-square of an AC voltage component Vᵣₘₛ=50 [mV] to the photoelectric conversion element while changing its frequency from 1.0×10⁻² [Hz] to 1.0×10⁶ [Hz],
in a plot in which a horizontal axis represents a frequency [Hz] and a vertical axis represents a phase [deg] based on the measurement results of the impedance,
the phase of the impedance has a maximum value in a low frequency range of 1.0×10⁻² to less than 1.0×10² [Hz],
and the phase of the impedance has a maximum value in a high frequency range of 1.0×10² to 1.0×10⁶ [Hz];
and in a Nyquist plot in which a horizontal axis represents an impedance real part Z' [Ω] and a vertical axis represents an impedance imaginary part Z" [Ω] based on the measurement results of the impedance,
the maximum value R_{rec} [Ω] out of resistance values obtained by fitting an arc corresponding to the maximum value of the phase in the low frequency range with a parallel circuit of a resistance element and a constant phase element satisfies the following formula (E1): $1.0 \times {10}^{4} \leq {R}_{rec} \times S \leq 1.0 \times {10}^{7}$
and the maximum value R_{ct} [Ω] out of resistance values obtained by fitting an arc corresponding to the maximum value of the phase in the high frequency range with the parallel circuit of the resistance element and the constant phase element, and the R_{rec} [Ω] satisfy the following formula (E2). ${R}_{rec} / {R}_{ct} \geq 25$

As a result of investigations made by the inventors of the present invention, it has been found that the deterioration of the photoelectric conversion element during durable use resulting from a defect in the photoelectric conversion layer can be suppressed by satisfying the above-mentioned configuration. In the related art, when the resistance of a photoelectric conversion element is increased to suppress its deterioration, the movement of charge generated in the photoelectric conversion layer is hindered, and its initial photoelectric conversion efficiency deteriorates. In contrast, when the resistance is lowered to improve the initial photoelectric conversion efficiency, a deterioration-suppressing effect becomes insufficient instead.

In view of the foregoing, the inventors of the present invention have found that initial photoelectric conversion efficiency and the suppression of deterioration can both be achieved when the ratio of an impedance at low frequencies to an impedance at high frequencies satisfies R_{rec}/R_{ct}≥25 under the condition that the impedance at low frequencies satisfies the following formula (E1) $1.0 \times {10}^{4} \leq {R}_{rec} \times S \leq 1.0 \times {10}^{7}$ The inventors of the present invention have presumed the reason for the foregoing to be as described below.

On one hand, the impedance R_{rec} [Ω] at low frequencies refers to a recombination resistance that correlates with the difficulty of the recombination of an electron and a hole generated in the photoelectric conversion layer, and when the value is a certain value or more, durability deterioration is suppressed. However, when the value is too large, the initial photoelectric conversion efficiency deteriorates. It is required to satisfy the following formula (E1) to strike a balance between the suppression and the efficiency. $1.0 \times {10}^{4} \leq {R}_{rec} \times S \leq 1.0 \times {10}^{7}$

On the other hand, the impedance R_{ct} [Ω] at high frequencies refers to a charge transfer resistance that correlates with the ease of the movement of charge when the photoelectric conversion element performs photoelectric conversion, and as the value becomes smaller, the initial photoelectric conversion efficiency is improved. Accordingly, it is required to satisfy the following formula (E2) in addition to the formula (E1) to achieve both the initial photoelectric conversion efficiency and the suppression of the deterioration. ${R}_{rec} / {R}_{ct} \geq 25$

As described above, the related-art difficulty in achieving both the initial photoelectric conversion efficiency and the suppression of the deterioration can be essentially solved by providing a condition that gives a correlation between impedance characteristic values in different frequency ranges as in the formula (E2), rather than only the condition for a range for a single impedance characteristic value as in the formula (E1), or the condition for a range for each of a plurality of impedance characteristic values.

The effects of the present invention can be achieved when the respective configurations synergistically exert effects on each other as in the mechanism as described above.

The present invention is described in detail below by way of preferred embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

### [Impedance Characteristics of the Present Invention]

The photoelectric conversion element of the present invention is a photoelectric conversion element including: a first electrode; a second electrode; and a photoelectric conversion layer arranged between the first electrode and the second electrode, the layer containing a crystal having a perovskite structure, the element being characterized in that:
when the smaller one of the electrode area of the first electrode and the electrode area of the second electrode is represented by S [cm²],
and an impedance is measured by applying an AC voltage with a DC voltage component V_{DC}=0 [V] and a root-mean-square of an AC voltage component Vᵣₘₛ=50 [mV] to the photoelectric conversion element while changing its frequency from 1.0×10⁻² [Hz] to 1.0×10⁶ [Hz],
in a plot in which a horizontal axis represents a frequency [Hz] and a vertical axis represents a phase [deg] based on the measurement results of the impedance,
the phase of the impedance has a maximum value in a low frequency range of 1.0×10⁻² to less than 1.0×10² [Hz],
and the phase of the impedance has a maximum value in a high frequency range of 1.0×10² to 1.0×10⁶ [Hz]; and
in a Nyquist plot in which a horizontal axis represents an impedance real part Z' [Ω] and a vertical axis represents an impedance imaginary part Z" [Ω] based on the measurement results of the impedance,
the maximum value R_{rec} [Ω] out of resistance values obtained by fitting an arc corresponding to the maximum value of the phase in the low frequency range with a parallel circuit of a resistance element and a constant phase element satisfies the following formula (E1): $1.0 \times {10}^{4} \leq {R}_{rec} \times S \leq 1.0 \times {10}^{7}$
and the maximum value R_{ct} [Ω] out of resistance values obtained by fitting an arc corresponding to the maximum value of the phase in the high frequency range with the parallel circuit of the resistance element and the constant phase element, and the R_{rec} [Ω] satisfy the following formula (E2): ${R}_{rec} / {R}_{ct} \geq 25$

Herein, the plot in which the horizontal axis represents a frequency [Hz] and the vertical axis represents a phase [deg] is a graph in which the frequency values of the AC voltage in the impedance measurement and angles "arctan(Z"/Z')" formed by the real part Z' and imaginary part Z" of the impedance obtained when the frequency is applied on a complex plane are associated with each other as shown in Fig. 1.

In addition, the Nyquist plot in which the horizontal axis represents an impedance real part Z' [Ω] and the vertical axis represents an impedance imaginary part Z" [Ω] corresponds to a graph in which the above-mentioned real part Z' and imaginary part Z" of the impedance are plotted on the complex plane as shown in Fig. 2.

However, in the present invention, for the positive and negative signs, -Z" is adopted as the vertical axis. Correspondingly, in the present invention, for the phase [deg], the - (minus) direction is also adopted as the vertical axis. Accordingly, the maximum value of the phase in the low frequency range or high frequency range in the present invention means an extreme value at which the phase increases in the - direction.

Those problems of positive and negative signs depend on the definition of a complex impedance Z [Ω] and are not essential problems.

The meaning of the arc corresponding to the maximum value of the phase in the low frequency range or high frequency range is described. As shown in Fig. 3(a), when the maximum value in the low frequency range is represented by θ_{low}, the θ_{low} corresponds to a slope when a tangent line passing through the origin is drawn to an arc existing in the low frequency range in the Nyquist plot of Fig. 3(b). Accordingly, the arc corresponding to the maximum value of the phase refers to precisely the arc to which such tangent line is drawn.

The constant phase element of the present invention is described. The constant phase element is used in an equivalent circuit for fitting impedance data illustrated as an example in Fig. 4, and is indicated by "CPE (1 to 3)" appearing in the R-CPE parallel circuit of Fig. 4. The constant phase element is an element in which a capacitive element (capacitor element) is mathematically modified for fitting. The complex impedance of the capacitive element is represented by the following formula (E7): $ZC = 1 / \left(i2πfC\right)$ (provided that "i" represents an imaginary unit, π represents the ratio of the circumference of a circle to its diameter, "f" represents a frequency [Hz], and C represents a capacitance [F]).
The complex impedance of the constant phase element is represented by the following formula (E8). $ZCPE = 1 / \left[\left(i2πf\right)PT\right]$

The formula (E8) matches the formula (E7) when P=1 is satisfied (in this case, T=C is satisfied), and hence the constant phase element is certainly a mathematical modification of the capacitive element.

The exponent P of the constant phase element represented by the formula (E8) corresponds to the degree of collapse of an arc in a Nyquist plot. P=1 is satisfied when the arc is a perfect semicircle, but P<1 is satisfied when the arc is a semicircle submerged in the fourth quadrant on the Nyquist plot. The foregoing means that impedance dispersion is larger as P is smaller than 1 because the degree of collapse of the arc on the Nyquist plot correlates with the impedance dispersion.

In order to enhance the effects of the present invention, it is more preferred that the product "R_{rec}×S" [Ω·cm²] satisfy the following formula (E5). $1.0 \times {10}^{5} < {R}_{rec} \times S < 1.0 \times {10}^{6}$

In addition, it is preferred that the ratio "R_{rec}/R_{ct}" satisfy the following formula (E6). ${R}_{rec} / {R}_{ct} \geq 50$

In addition, in order to suppress the deterioration more effectively, it is preferred that the exponent P_{rec} of the constant phase element obtained by the fitting performed to obtain the R_{rec} [Ω] satisfy the following formula (E3). ${P}_{rec} \geq 0.80$ When the formula (E3) is satisfied, impedance dispersion at low frequencies corresponding to a recombination resistance is sufficiently small. Accordingly, the probability of concealing a defect of the photoelectric conversion layer is improved because the recombination resistance contributing to the concealment of the defect of the photoelectric conversion layer becomes uniform with respect to the plane direction of the photoelectric conversion layer.

In addition, in order to effectively maintain high initial photoelectric conversion efficiency, it is preferred that the exponent P_{ct} of the constant phase element obtained by the fitting performed to obtain the R_{ct} [Ω] satisfy the following formula (E4). ${P}_{ct} \leq 0.95$ When the formula (E4) is satisfied, impedance dispersion at high frequencies corresponding to a charge transfer resistance is sufficiently large. Accordingly, a high-resistance recombination resistance element that contributes to global defect concealment with respect to the plane direction of the photoelectric conversion layer and a low-resistance charge transfer resistance element that contributes to a local charge transfer path of the photoelectric conversion element further coexist because a low-resistance path for the movement of photo-charge generated in the photoelectric conversion layer is sufficiently present.

The photoelectric conversion element of the present invention and the configuration of each layer thereof are described in detail below with reference to preferred embodiments. Several examples for obtaining the above-mentioned section [Impedance Characteristics of the Present Invention] are described below. Accordingly, the present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which the concentration of an element to be incorporated gradually changes, or a layer that may form a complicatedly intricate structure together with another layer. In addition, the elemental analysis of the layer may be performed by, for example, performing the TOF-SIMS/FE-TEM/EDS line analysis measurement of a cross section of the photoelectric conversion element and observing the element distribution of a specific element. The analysis of each layer may be performed by peeling and removing a layer from a completed photoelectric conversion element to expose the layer to be analyzed. For the quantification of a volume ratio, the area ratio of an exposed surface or cross section may also be used as the volume ratio of the layer.

Fig. 5 is a sectional view for schematically illustrating the configuration of the photoelectric conversion element according to one embodiment of the present invention. A photoelectric conversion element 1 includes a substrate 2, and a second electrode 3, an electron-transporting layer 4, a photoelectric conversion layer 5, a charge-transporting layer 6, and a first electrode 7 arranged thereon. One of the first electrode 7 and the second electrode 3 is an anode, and the other is a cathode. A current can be extracted by connecting the first electrode 7 and the second electrode 3 with an external circuit.

The photoelectric conversion layer 5 is excited by light that has entered the layer through the substrate 2, the second electrode 3, and the electron-transporting layer 4, or the first electrode 7 and the charge-transporting layer 6 to generate an electron or a hole. That is, the photoelectric conversion layer 5 generates a current between the first electrode 7 and the second electrode 3. The electron-transporting layer 4 is a layer arranged between the photoelectric conversion layer 5, and the two electrodes 3 and 7, and may not be formed in some cases. A form in which the plurality of electron-transporting layers 4 and photoelectric conversion layers 5 are laminated may be adopted. Such form may also be referred to as "tandem structure." The respective members are described below. In addition, the photoelectric conversion element may be produced in the order of the first electrode 7, the charge-transporting layer 6, the photoelectric conversion layer 5, the electron-transporting layer 4, and the second electrode 3 on the substrate 2.

### [Photoelectric Conversion Element]

The photoelectric conversion element of the present invention is characterized by including: the first electrode; the second electrode; the photoelectric conversion layer arranged between the first electrode and the second electrode, the photoelectric conversion layer containing the crystal having a perovskite structure; and the charge-transporting layer between the photoelectric conversion layer and the first electrode. In addition, in order to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a perovskite crystal in its photoelectric conversion layer.

A method of forming each of the layers including the photoelectric conversion layer and charge-transporting layer of the photoelectric conversion element of the present invention is, for example, a coating method or a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with each layer.

The respective layers are described below.

### [Substrate]

The photoelectric conversion element 1 of the present invention may include the substrate 2, and examples thereof include a transparent glass substrate made of soda-lime glass or alkali-free glass, a ceramic substrate, and a transparent plastic substrate. When light is taken in from the first electrode 7 side, an opaque material may be used as the substrate 2, and when light is taken in from the second electrode 3 side, the substrate 2 is formed of a transparent material.

### [Electrode]

The photoelectric conversion element of the present invention includes the first electrode and the second electrode. A material for the first electrode 7 or the second electrode 3 is not particularly limited, and a material that has hitherto been known may be used. Examples thereof include: metals, such as gold, silver, titanium, and copper; sodium; a sodium-potassium alloy; lithium; magnesium; carbon; a carbon nanotube; aluminum; a magnesium-silver mixture; a magnesium-indium mixture; an aluminum-lithium alloy; an Al/Al₂O₃ mixture; and an Al/LiF mixture. Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers. Those materials may be used alone or in combination thereof. At least one electrode of the first electrode 7 or the second electrode 3 on a light incident side is a transparent electrode, and the other may be a transparent electrode or may also serve as a reflective layer formed of a light reflective material, or may be a transparent electrode including a reflective layer on a side opposite to the light incident side. When the first electrode 7 is on the light incident side, the second electrode 3 and the substrate 2 may be a transparent electrode and a reflective layer, respectively. The electrode may be a patterned electrode.

### [Photoelectric Conversion Layer]

The photoelectric conversion layer 5 contains the crystal having a perovskite structure. The crystal having a perovskite structure to be used in the present invention is preferably represented by the following general formula [1].

ABX₃ [1]

In the general formula [1], A represents a monovalent cation of an organic molecule or a metal atom, B represents a divalent metal cation, and X represents a monovalent halide anion.

A in the general formula [1] preferably represents CₚN_{q}Hᵣ ("p", "q", and "r" each represent a positive integer) in the case of, for example, the organic molecule. Specific examples thereof include methylammonium and formamidinium.

In addition, an inorganic atom is not particularly limited, and lithium, cesium, sodium, potassium, and rubidium are preferred. Those organic molecules or inorganic atoms may be used alone or in combination thereof.

When the cation A to be included is too large to fit in a crystal having a three-dimensional perovskite structure, a crystal having a two-dimensional perovskite structure, a crystal having a 2.5-dimensional perovskite structure with properties of both the two-dimensional and three-dimensional perovskite structures, a two-layer crystal having three-dimensional and two-dimensional perovskite structures, or a crystal having a mixed three-dimensional/two-dimensional perovskite structure is formed, and any of the structures functions as the photoelectric conversion layer. The two-layer crystal having three-dimensional and two-dimensional perovskite structures refers to a crystal in which the crystals having three-dimensional and two-dimensional perovskite structures are laminated as independent and separate layers. The crystal having a mixed three-dimensional/two-dimensional perovskite structure refers to a crystal having a structure in which both the regions or domains of crystals having two-dimensional or 2.5-dimensional layered and three-dimensional perovskite structures are mixed.

It is preferred that the crystal having a two-dimensional perovskite or 2.5-dimensional perovskite structure be represented by each of the following general formulae [2] to [4] ("n" represents a positive integer).

R'₂Aₙ₋₁BₙX₃ₙ₊₁ [2]

R"Aₙ₋₁BₙX₃ₙ₊₁ [3]

R‴AₙBₙX₃ₙ₊₁ [4]

The general formula [2], the general formula [3], and the general formula [4] form perovskite structures of a Ruddlesden-Popper (RP) type, a Dion-Jacobson (DJ) type, and an Alternating cations in the interlayer (ACI) type, respectively.

R', R", and R‴ in the general formulae [2] to [4] each represent a cation of an organic molecule or a metal that may have a substituent. Specifically, ethylammonium, propylammonium, n-butylammonium, n-hexylammonium, n-octylammonium, 1,6-hexadiammonium, iso-butylammonium, 3-(nonafluoro-tert-butyloxy)propylamine, 1,3-propanediammonium, 1,5-pentamethylenediamine, octyldiammonium, 2,2-(ethylenedioxy)bis(ethylammonium), 5-aminovaleric acid, 4-tert-butylammonium, N,N'-dimethylethylene-1,2-diammonium, 2,2,3,3,3-pentafluoropropylammonium, guanidinium, propylammonium, propargylamine, an alkylammonium, cyclohexylmethylammonium, 4-(aminomethyl)piperidinium, piperidinium, pyrrolidinium, cyclohexylammonium, 4-fluorophenethylammonium, 4-fluorophenethylammonium, trifluoromethylbenzylammonium, pentafluorobenzylammonium, pentafluorophenylethylammonium, 4-methoxyphenethylammonium, imidazolium, pyridinium, 3-thiophenemethylammonium, 2-thiopheneethylammonium, 2-thiopheneformamidinium, 2-thiophenemethylammonium, 1-naphthylmethylammonium, 2-naphthylmethylammonium, phenethylammonium, phenylammonium, benzylammonium, 2,5-thiophenedimethylammonium, phenylpropylammonium, 1,4-phenylenedimethanamine, 3-phenyl-2-propen-1-ammonium, phenylbutylammonium, 4-tert-butylbenzylammonium, 3-(aminomethyl)piperidinium, and 4-(aminomethyl)piperidinium are preferred.

B in each of the general formulae [1] to [4] represents a metal atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, and bismuth are preferred from the viewpoint of the overlap of electron orbits. Those metal atoms may be used alone or in combination thereof.

X in each of the general formulae [1] to [4] represents a halogen atom, and examples thereof include chlorine, bromine, and iodine. Those halogen atoms may be used alone or in combination thereof. Of those, a halogen atom is preferred because, when the halogen atom is incorporated into the structure, the above-mentioned crystal having a perovskite structure easily becomes soluble in an organic solvent, and hence the application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbI_{α}Br_{3-α}, MAPbI_{α}Cl_{3-α}, Cs_{0.05}(MA_{0.17}FA_{0.83})_{0.95}Pb(I_{0.83}Br_{0.17})₃, {Cs_{β1}(FA_{β2}MA_{1-β2})_{1-β1}}_{γ1}Pb(I_{β3}Br_{1-β3})_{γ2}, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Cs_{β}(MA)_{1-β}PbI₃, Cs_{β}(FA)_{1-β}PbI₃, MA_{β}(FA)_{1-β}PbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (4FPEA)₂ (FA_{0.3}MA_{0.7})₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (TTDMA)MA₃Pb₄I₁₃, (TTDMA)MA₄Pb₅I₁₆, (BA_{0.9}PEA_{0.1})₂MA₄Pb₅I₁₆, (BA_{0.9}PEA_{0.1})₂MA₃Pb₄I₁₃, (4FPEA)₂MA₃Pb₄I₁₃, (4FPEA)₂MA₄Pb₅I₁₆, (BA)₂MA₂Pb₃I₁₀, (BA)₂MA₃Pb₄I₁₃, (TEA)₂MA₂Pb₃I₁₀, (BA)₂MA₄Pb₅I₁₆, (BA)₂MA₃Pb₄I₁₃, CsSnBr₃, CsSnI₃, FA_{0.75}MA_{0.25}Sn_{0.95}Ge_{0.05}I₃, FAMASnGeI₃, FASnBr₃, FASnI₃, MA₂Sn₃I₈, MASnBr₃, MASnGeI₃, and MASnI₃ are preferred. The A site, B site, or X site of each of the general formulae may be adjusted to be deficient or excessive in accordance with purposes, and the combinations of β1 to β3, and γ1 and γ2 may be changed in accordance with purposes. Examples of the combinations of β1 to β3, and γ1 and γ2 are as shown in Table 1. Particularly preferred ranges are 0.03≤β1≤0.10, 0.80≤β2≤0.96, 0.80≤β3≤0.96, 0.95≤γ1≤1.05, and 2.95≤γ2≤3.05. In addition, the ranges of α and β are 0<α<3 and 0<β<1, respectively. MACl may be incorporated as a material for forming a perovskite crystal.

### [Table 1]

**Table 1**

| β1 | β2 | 1-β2 | β3 | 1-β3 | γ1 | γ2 |
|---|---|---|---|---|---|---|
| 0.05 | 0.83 | 0.17 | 0.83 | 0.17 | 1.00 | 3.00 |
| 0.05 | 0.83 | 0.17 | 0.83 | 0.17 | 0.99 | 2.99 |
| 0.05 | 0.83 | 0.17 | 0.83 | 0.17 | 0.98 | 2.98 |
| 0.05 | 0.83 | 0.17 | 0.83 | 0.17 | 0.97 | 2.97 |
| 0.05 | 0.83 | 0.17 | 0.83 | 0.17 | 0.96 | 2.96 |
| 0.05 | 0.83 | 0.17 | 0.83 | 0.17 | 1.01 | 3.01 |
| 0.05 | 0.83 | 0.17 | 0.83 | 0.17 | 1.02 | 3.02 |
| 0.05 | 0.83 | 0.17 | 0.83 | 0.17 | 1.03 | 3.03 |
| 0.05 | 0.83 | 0.17 | 0.83 | 0.17 | 1.04 | 3.04 |
| 0.05 | 0.83 | 0.17 | 0.95 | 0.05 | 1.00 | 3.00 |
| 0.05 | 0.83 | 0.17 | 0.95 | 0.05 | 0.97 | 2.97 |
| 0.05 | 0.83 | 0.17 | 0.95 | 0.05 | 0.98 | 2.98 |
| 0.05 | 0.83 | 0.17 | 0.95 | 0.05 | 0.99 | 2.99 |
| 0.05 | 0.83 | 0.17 | 0.95 | 0.05 | 1.01 | 3.01 |
| 0.05 | 0.83 | 0.17 | 0.95 | 0.05 | 1.02 | 3.02 |
| 0.05 | 0.83 | 0.17 | 0.95 | 0.05 | 1.03 | 3.03 |

In the above-mentioned specific examples, "MA" represents methylammonium, "FA" represents formamidinium, "PEA" represents phenethylammonium, "PTA" represents phenyltriethylammonium, "ThMA" represents 2-thiophenemethylammonium, "3BBA" represents 3-bromobenzylammonium, "3AMPY" represents 3-(aminomethyl)pyridine, "PDMA" represents 1,4-phenylenedimethanammonium, "TTDMA" represents thieno[3,2-b]thiophene-2,5-diyldimethanammonium, "4FPEA" represents 4-fluorophenethylammonium, "BA" represents butylammonium, and "TEA" represents 2-thiopheneethylammonium.

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element is improved.

An organic-inorganic perovskite compound to be used in the present invention is preferably a crystalline semiconductor. The term "crystalline semiconductor" means a semiconductor that enables the measurement of an X-ray scattering intensity distribution to detect a scattering peak. When the organic-inorganic perovskite compound is the crystalline semiconductor, the mobility of the electron in the organic-inorganic perovskite compound increases, and the photoelectric conversion efficiency of the photoelectric conversion element is improved.

The thickness of the photoelectric conversion layer according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm or more, a more preferred upper limit is 1,200 nm, a still more preferred lower limit is 100 nm, and a still more preferred upper limit is 1,000 nm.

### [Charge-transporting Layer]

In the present invention, the photoelectric conversion element includes the charge-transporting layer between the photoelectric conversion layer and the first electrode, and it is preferred that the charge-transporting layer contain a charge-transporting material and an insulating resin on the surface of the photoelectric conversion layer. When the charge-transporting layer is a mixture system of the charge-transporting material and the insulating resin, it becomes easier to achieve both of a local low-resistance charge transfer resistance element contributing to the initial photoelectric conversion efficiency and a global high-resistance element contributing to the suppression of the deterioration. From the viewpoint, it is preferred that the insulating resin have a volume specific resistivity of 10⁸ Ω·cm or more.

The inventors of the present invention have presumed the preferred configuration of the charge-transporting layer as means for achieving the configuration of the present invention as described above and reasons therefor to be described below.

On one hand, the impedance R_{rec} [Ω] at low frequencies corresponds to an interfacial resistance between the photoelectric conversion layer and the charge-transporting layer because the impedance means a recombination resistance that correlates with the difficulty of the recombination of an electron and a hole generated in the photoelectric conversion layer. To set the value to a certain value or more and suppress durable deterioration, a defect of the photoelectric conversion element only needs to be sufficiently concealed with the insulating resin of the charge-transporting layer. However, when the value is too large, charge transfer at the interface is hindered, and the initial photoelectric conversion efficiency deteriorates. As described above, it has been required to satisfy the following formula (E1) to strike a balance between the suppression and the efficiency. $1.0 \times {10}^{4} < {R}_{rec} \times S < 1.0 \times {10}^{7}$

On the other hand, the impedance R_{ct} [Ω] at high frequencies means a charge transfer resistance that correlates with the ease of the movement of a charge when the photoelectric conversion element performs photoelectric conversion, and the impedance corresponds to the bulk resistance of the charge-transporting layer. The charge-transporting material of the charge-transporting layer is effective in setting the value small to make the charge movement of the photoelectric conversion element smooth and improve the initial photoelectric conversion efficiency.

From the foregoing, when the charge-transporting layer is a mixture system of an insulating resin contributing to the increase of the R_{rec} and a charge-transporting material contributing to the reduction of the R_{ct}, it becomes easier to obtain a photoelectric conversion element that satisfies the following formula (E2) of the present invention, and achieves both the initial photoelectric conversion efficiency and the suppression of the deterioration. ${R}_{rec} / {R}_{ct} \geq 25$

In addition, in the present invention, the charge-transporting material is preferably a charge-transporting particle. When the charge-transporting material is a particle, impedance dispersion at high frequencies corresponding to the bulk resistance becomes larger, and it becomes easier to satisfy the following formula (E4). ${P}_{ct} \leq 0.95$ As described above, when the formula (E4) is satisfied, impedance dispersion at high frequencies corresponding to the charge transfer resistance becomes sufficiently larger, and a low-resistance path for the movement of photo-charge generated in the photoelectric conversion layer is sufficiently present in the charge-transporting layer. As a result, it becomes even easier for a high-resistance recombination resistance element contributing to global defect concealment with respect to the plane direction of the photoelectric conversion layer and a low-resistance charge transfer resistance element contributing to a local charge transfer path of the photoelectric conversion element to coexist.

Further, the charge-transporting material is more preferably a pigment having a particle size of 10 to 500 nm. A charge distribution capable of suppressing migration is easily formed in the above-mentioned range. In addition, specific examples of the pigment include a phthalocyanine pigment, an azo pigment, a lake pigment, a quinacridone pigment, a dioxazine pigment, a perylene pigment, and an isoindolinone pigment.

The volume ratio of the charge-transporting material to the insulating resin is preferably 5 to 30 times. When the volume ratio falls within the range, it becomes easier to satisfy the following formula (E3). ${P}_{rec} \geq 0.80$ As described above, when the formula (E3) is satisfied, impedance dispersion at low frequencies corresponding to the recombination resistance becomes sufficiently small, and the recombination resistance contributing to the concealment of a defect of the photoelectric conversion layer becomes uniform with respect to the plane direction of the photoelectric conversion layer. Accordingly, the probability of concealing a defect of the photoelectric conversion layer is improved, and it becomes easier to achieve both the initial photoelectric conversion efficiency and the suppression of the deterioration. The ratio between the volume of the insulating resin and the volume of a charge-transporting substance may be determined from, for example, the area ratio of a cross section determined by FETEM/EDS as described above.

The charge-transporting material is more preferably a phthalocyanine compound, and the charge-transporting material still more preferably has a structure represented by the following formula (Pc-2). The charge-transporting material makes it easier to increase the impedance dispersion at high frequencies corresponding to the bulk resistance, and the achievement of both the initial photoelectric conversion efficiency and the suppression of the deterioration becomes easier.

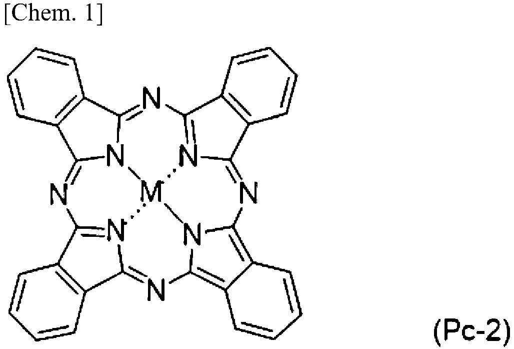

M in the formula (Pc-2) represents H₂ or a metal atom that may have a ligand. In the present invention, the structure of a chemical substance may be determined by nuclear magnetic resonance (NMR).

In particular, when M in the formula (Pc-2) represents H₂, the formula (Pc-2) is represented by the following formula (Pc-1).

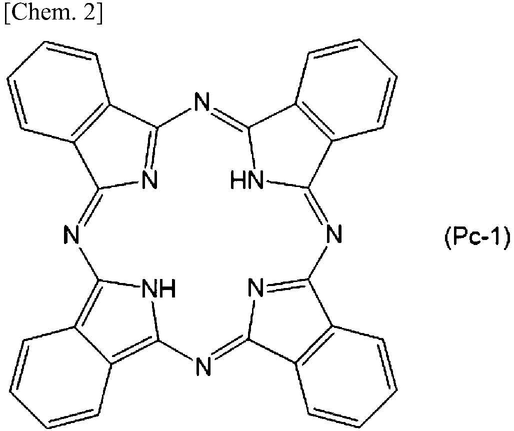

Specific examples of the insulating resin include a polyacetal resin, an acrylic resin, a polyarylate resin, a polycarbonate resin, a polyvinyl acetate resin, a polyester resin, a polyamide resin, a polyurethane resin, and a polystyrene resin.

The insulating resin more preferably has a glass transition temperature of 95°C or less. When the glass transition temperature falls within the range, the insulating resin is easily brought into dense contact with the charge-transporting material, and the coexistence of a local low-resistance element contributing to the initial photoelectric conversion efficiency and a global high-resistance element contributing to the suppression of the deterioration can be more effectively achieved. Accordingly, it becomes easier to obtain the impedance parameters of the present invention. The glass transition temperature may be determined by differential scanning calorimetry (DSC).

The insulating resin is more preferably a polyvinyl acetal resin or a polyvinyl butyral resin. When the insulating resin is adopted, the insulating resin is easily brought into dense contact with the charge-transporting material, and the impedance parameters of the present invention can be more effectively obtained.

The charge-transporting layer more preferably contains an aromatic ring compound having a hydroxy group, the compound being different from the charge-transporting material and the insulating resin. When the charge-transporting layer contains the aromatic ring compound having a hydroxy group, the charge-transporting material and the insulating resin can be more easily brought into contact with each other, and the impedance parameters of the present invention can be more effectively obtained.

The photoelectric conversion element may include a second charge-transporting layer between the first electrode and the charge-transporting layer. When the photoelectric conversion element includes the second charge-transporting layer, the transfer of carriers to an electrode may be facilitated.

The thickness of the charge-transporting layer is preferably 1 to 1,000 nm, more preferably 5 to 500 nm, particularly preferably 10 to 200 nm.

The charge-transporting layer may be formed by preparing a coating liquid for a charge-transporting layer containing the above-mentioned respective materials and a solvent, forming a coating film of the liquid on the photoelectric conversion layer, and drying the coating film. Examples of the solvent to be used for the coating liquid include an alcohol-based solvent, a ketone-based solvent, an ether-based solvent, an ester-based solvent, and an aromatic hydrocarbon-based solvent. Of those solvents, an alcohol-based solvent or an aromatic hydrocarbon-based solvent is preferred.

### [Second Charge-transporting Layer]

In the present invention, the photoelectric conversion element 1 may further include the second charge-transporting layer between the charge-transporting layer 6 and the first electrode 7 from the viewpoint of the compatibility of a film of the charge-transporting layer 6.

A material for the second charge-transporting layer is not particularly limited, and examples thereof include a spirofluorene compound, a triphenylamine compound, a chrysene compound, a pyrene compound, a phthalocyanine compound, a carbazole compound, a fluorene compound, a phenylcyclohexane compound, a benzidine compound, a phenoxazine compound, a phenylenediamine compound, a thiocyanate compound, and a thiophene compound. The compound particularly preferably has an aromatic ring from the viewpoint of the compatibility of a film interface, and Spiro-OMeTAD, PTAA, or a phthalocyanine compound is preferred.

In addition, the second charge-transporting layer may contain a dopant as an additive in order to improve its charge transportation capability. Examples of a substance that may be used as the dopant include lithium compounds such as lithium bis(trifluoromethanesulfonyl)imide, cobalt compounds such as [tris(2-(1H-pyrazol-1-yl)-4-tert-butylpyridine)cobalt(III) tris(bis(trifluoromethylsulfonyl)imide)], boron compounds such as tetrakis(pentafluorophenyl)borate, molybdenum compounds such as tris[1-(methoxycarbonyl)-2-(trifluoromethyl)-ethane-1,2-dithiolene]molybdenum, organic compounds each having a tetracyanoquinodimethane skeleton such as 2,3,4,6-tetrafluoro-7,7,8,8-tetracyanoquinodimethane, and organic compounds each having a pyridine skeleton such as 4-tert-butylpyridine.

### [Control of Particle Size of Charge-transporting Particle]

The particle size of the charge-transporting particle may be changed by dispersing the coating liquid for a charge-transporting layer with a paint shaker, and the particle size may be reduced by increasing a dispersion time. In addition, the particle size may be reduced by further subjecting the coating liquid for a charge-transporting layer to a centrifuge.

### [Electron-transporting Layer]

In the photoelectric conversion element of the present invention, the electron-transporting layer 4 may be arranged between the second electrode 3 and the photoelectric conversion layer 5 as illustrated in Fig. 5.

A material for the electron-transporting layer 4 is not particularly limited, and examples thereof include an N-type conductive polymer, an N-type low-molecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthroline, hydroxyquinolinatoaluminum, an oxadiazole compound, a benzimidazole compound, a naphthalenetetracarboxylic acid compound, a fullerene compound, a perylene derivative, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide. Specifically, tin oxide may be obtained by causing tin(II) chloride, tin(IV) chloride, tin(II) chloride dihydrate, or tin(IV) chloride pentahydrate to react with oxygen.

A preferred lower limit of the thickness of the electron-transporting layer 4 is 1 nm, and a preferred upper limit thereof is 2,000 nm. When such thickness is 1 nm or more, a hole can be sufficiently blocked, and when the thickness is 2,000 nm or less, the electron-transporting layer 4 is less liable to serve as a resistance at the time of the electron transportation, and hence the photoelectric conversion efficiency is improved. A more preferred lower limit of the thickness is 3 nm, a more preferred upper limit thereof is 1,000 nm, a still more preferred lower limit thereof is 5 nm, and a still more preferred upper limit thereof is 500 nm.

### [Photoelectric Conversion Apparatus]

A photoelectric conversion apparatus may be formed by using the plurality of photoelectric conversion elements of the present invention. When the plurality of photoelectric conversion elements are connected, such photoelectric conversion apparatus may also be referred to as "photoelectric conversion cell" or "photoelectric conversion module." In the photoelectric conversion element, elements having different absorption wavelengths may be laminated to increase an output voltage. In addition, the photoelectric conversion apparatus includes the photoelectric conversion element of the present invention and an inverter. The inverter may be a converter for converting a DC voltage to an AC voltage. The photoelectric conversion apparatus may include an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor. In order to impart a function of, for example, maintaining or increasing the amount of incident light, a surface layer to which water or dirt is hard to adhere, or a function of collecting or guiding light may be added.

### [Moving Body]

Fig. 6 is a perspective view for schematically illustrating a moving body including the photoelectric conversion element according to one embodiment of the present invention. A moving body 30 includes a photoelectric conversion element 31 of the present invention and a body 32 including the photoelectric conversion element 31. The photoelectric conversion element 31 is arranged on the position of the body 32 at which ambient light can be received. When the moving body 30 is an automobile, the photoelectric conversion element 31 may be arranged on a roof. Electric energy obtained by the photoelectric conversion element 31 may serve as the power of the moving body 30 or the power of any other electric equipment. Electric energy generated from the power of the moving body 30 may be used for the power of the photoelectric conversion element 31. When the moving body 30 is an automobile, friction energy generated with a brake may be converted into electric energy to be used for the control of the photoelectric conversion element 31.

The moving body 30 may be, for example, an automobile, a motorcycle, a railway vehicle, a ship, or a flying body including an artificial satellite, an airplane, and a drone. The configuration of the body 32 of the moving body 30 is not particularly limited, but is preferably formed of a material having high strength.

### [Building Material]

Fig. 7 is a perspective view for schematically illustrating a building material including the photoelectric conversion element according to one embodiment of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a thermal conductivity higher than that of the photoelectric conversion element 41. When the building material 40 is used for a roof or the like, the temperature of the photoelectric conversion element 41 may be increased by sunlight, and hence the photoelectric conversion efficiency may be reduced. The reduction of the photoelectric conversion efficiency can be suppressed by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, or may show the same color. The exterior 44a and the exterior 44b may be formed of the same member, or may be formed of different members. A paint or a transparent substrate may be used as each of the exteriors. An exterior having small light absorption and a high heat-shielding property is preferred.

In addition to the application examples described above, the following application examples may be given: portable devices, such as a calculator, a sensor, and a small solar panel; wearable devices, such as a glasses-type terminal, a watch-type terminal, and a portable medical device; sheet structures supported by a plurality of frames, such as a tent, a plastic house, and a loading platform of a truck; and structures to be used by being fixed, such as a road surface panel, a floating panel, a building material utilizing the flexibility of a substrate, a wall-type building material, a glass-type building material, and a mega solar panel.

### [Method of producing Photoelectric Conversion Element]

A method of producing the photoelectric conversion element of the present invention includes the steps of: forming a first electrode; forming a second electrode; and forming a photoelectric conversion layer containing a crystal having a perovskite structure between the first electrode and the second electrode.

The respective steps of the production method are described below.

### [Step of forming First Electrode and Step of forming Second Electrode]

The method of producing a photoelectric conversion element of the present invention includes the steps of: forming the first electrode; and forming the second electrode. In the step of forming the first electrode and the step of forming the second electrode, an appropriate method may be selected in accordance with a material of the first electrode and a material of the second electrode, respectively. Examples of such method include, but are not limited to, a sputtering method, a vacuum vapor deposition method, a vapor phase growth method (CVD method), and a spray pyrolysis deposition method (SPD method). The materials of the first electrode and the second electrode are as described above. When one or both of the first electrode and the second electrode are transparent electrodes, the thickness of each of the transparent electrodes is preferably 0.03 to 3 µm.

When a solar cell is produced, cutting processing may be performed for circuit formation between steps. Examples of the cutting processing include mechanical patterning and laser patterning.

### [Modularization Step]

An element formed up to the electrode may be sealed. A sealing method is, for example, sealing with a resin or sealing with a film. Examples of a material used for the sealing include silazane, silicone rubber, resins each having a siloxane skeleton, and glass.

In addition, hairline treatment may be applied to the surface of the sealed element from the viewpoint of the suppression of adhesion between elements occurring during winding in a roll-to-roll system.

### [Step of forming Photoelectric Conversion Layer]

The step of forming the photoelectric conversion layer may include a step of applying a liquid containing the material of the photoelectric conversion layer as described above. Examples of an application method include a spin coating method, a blade coating method, a slit die coating method, a screen printing method, a bar coater method, a casting method, a printing transfer method, a dip-up method, an ink jet method, a spray method, and a vacuum vapor deposition method. The method is appropriately selected therefrom in accordance with the properties of a photoelectric conversion layer to be produced, such as thickness control and orientation control.

Annealing treatment may be performed under reduced pressure or in an inert atmosphere (in a nitrogen or argon atmosphere) in order to remove a solvent or a dispersion medium from the applied liquid containing the material of the photoelectric conversion layer. The temperature of the annealing treatment is preferably 40 to 300°C, more preferably 50 to 150°C. The annealing treatment is preferably performed because materials for forming the respective layers may permeate each other at an interface between laminated layers to increase a contact area, and hence a short-circuit current can be increased.

### [Step of forming Charge-transporting Layer]

The method of producing a photoelectric conversion element of the present invention may include a step of forming a charge-transporting layer between the photoelectric conversion layer and the first electrode.

The step of forming the charge-transporting layer is preferably a method of applying a resin solution in which the insulating resin is dissolved. Thus, the insulating resin preferentially penetrates easily when there are gaps between perovskite crystal grains.

In addition, examples of the step of forming the charge-transporting layer include: a method including arranging a charge-transporting particle on the surface of the photoelectric conversion layer, and then applying the resin solution in which the insulating resin is dissolved; a method including applying the resin solution in which the insulating resin is dissolved on the surface of the photoelectric conversion layer, and then arranging the charge-transporting particle thereon; and a method including applying a solution, which is obtained by dispersing the charge-transporting particle in the resin solution in which the insulating resin is dissolved, on the surface of the photoelectric conversion layer.

### [Impedance Measurement and Analysis]

The measurement of an impedance is performed by: connecting the first electrode and second electrode of the photoelectric conversion element to an impedance analyzer through appropriate probes; and applying an AC voltage with a DC voltage component V_{DC}=0 [V] and a root-mean-square of an AC voltage component Vᵣₘₛ=50 [mV] to the photoelectric conversion element while changing its frequency from 1.0×10⁻² [Hz] to 1.0×10⁶ [Hz].

Various impedance analyzers may be used for the measurement, and for example, ModuLab XM MTS (manufactured by Solartron Analytical) may be used. Alternatively, SI1287 electrochemical interface (manufactured by Toyo Corporation) may be used as a power supply, Dielectric Interface Solartron 1296 (manufactured by Toyo Corporation) may be used as a current amplifier, Impedance/Gain-Phase Analyzer Solartron SI1260 (manufactured by Toyo Corporation) may be used as an ammeter, and Solartron Material Research and Test software Ver. 3.0.1 (manufactured by Solartron Analytical) may be used as measurement software.

Various kinds of analysis software may be used for the analysis of obtained data, and an example thereof is analysis software Zview Ver. 4 (manufactured by Scribner Associates, Inc.).

In the present invention, a plot in which a horizontal axis represents a frequency [Hz] and a vertical axis represents a phase [deg] based on the measurement results of an impedance (phase graph of a Bode plot), a Nyquist plot in which a horizontal axis represents an impedance real part Z' [Ω] and a vertical axis represents an impedance imaginary part Z" [Ω], and the like are analyzed. Specifically, with regard to the analysis of the Nyquist plot, when a two-axis graph including the real component Z' and imaginary component Z" of an impedance Z is created and represented by coordinates (Z', Z"), the result is fitted by using an appropriate equivalent circuit. At this time, the analysis is easily performed when an equivalent circuit in which at least two "resistance element-constant phase element parallel circuits (RCPE parallel circuits)" are connected in series is used in order to obtain a resistance (R) and the indices of constant phase elements (CPE) for each of a low frequency range (1.0×10⁻² to less than 1.0×10² [Hz]) and a high frequency range (1.0×10² to 1.0×10⁶ [Hz]). An example thereof is an equivalent circuit in which one series resistance (R1), one series inductance (L1), and three "resistance element-constant phase element parallel circuits" ("R4-CPE3", "R2-CPE1", and "R3-CPE2") are connected in series as illustrated in Fig. 4. The results of fitting a photoelectric conversion element 4 according to the present invention with the equivalent circuit of Fig. 4, which was performed in Example 4 of the present invention, are shown by a Nyquist plot in Fig. 2, but the impedance analysis of the present invention is not limited to the use of the equivalent circuit. In addition, the impedance parameters of the present invention may be obtained by another analysis method free of using an equivalent circuit, such as a method including extracting only a required arc on the Nyquist plot and performing fitting with one resistance element-constant phase element parallel circuit.

In the present invention, ModuLab XM MTS (manufactured by Solartron Analytical) was used for impedance measurement. The measurement conditions are as described below.

### <Measurement Conditions for ModuLab XM MTS>

Experiment type: Sample & Reference
Instrument configuration: Mat + Femto Ammeter
Ground: Internal
DC: 0 V
AC: 0.05 Vᵣₘₛ
Frequency: 1 MHz to 0.01 Hz
Number of measurement points: 6 points/decade

### Examples

The present invention is described in more detail below by way of Examples and Comparative Examples. The present invention is by no means limited to the following Examples without departing from the gist thereof. In the description of the following Examples, the term "part(s)" is by mass unless otherwise specified.

### <Production of Particle 1>

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm. After that, the temperature of the mixed liquid was increased to 200°C. Next, under a nitrogen flow atmosphere, the mixed liquid was subjected to a reaction at a temperature of 200°C for 4.5 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide a chlorogallium phthalocyanine particle in a yield of 71 mass%.

### Step (2)

4.65 Parts of the chlorogallium phthalocyanine particle was dissolved in 139.5 parts of concentrated sulfuric acid at a temperature of 10°C, and the solution was dropped into 620 parts of ice water under stirring so that the particle was reprecipitated, followed by filtration with a filter press under reduced pressure. At this time, No. 5C (manufactured by Advantec Toyo Kaisha, Ltd.) was used as a filter. The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times. Finally, the filter residue was freeze-dried to provide a hydroxygallium phthalocyanine particle (hydrous hydroxygallium phthalocyanine particle) having a solid content of 23 mass% in a yield of 71%. The hydroxygallium phthalocyanine particle was dried with a hyper-dry dryer (product name: HD-06R, frequency (oscillatory frequency): 2,455 MHz±15 MHz, manufactured by Biocon (Japan) Ltd.). Thus, a hydroxygallium phthalocyanine (HOGaPc) particle (crystal) having a water content of 1.0 mass% or less was obtained.

### Step (3)

5 Parts of the hydroxygallium phthalocyanine particle was mixed with 5 parts of an N-methylformamide solvent, and the mixture was subjected to dispersion treatment for 6 hours with a sand mill (TSG-1/4G-4U, manufactured by Igarashi Machine Production Co., Ltd. (currently AIMEX Co., Ltd.), disc diameter: 70 mm, number of discs: 5) containing 5 parts of glass beads, followed by filtration and drying to provide a particle 1 (specific gravity: 1.6).

### <Production of Resin Solution 1>

1.0 Gram of polyvinyl butyral (product name: BM-2, manufactured by Sekisui Chemical Co., Ltd., specific gravity: 1.6) was dissolved in 19 g of 2-propanol by stirring for 24 hours to provide a resin solution 1.

### (Production of Photoelectric Conversion Element)

### (Production of Photoelectric Conversion Element 1)

### [Formation of Electron-transporting Layer]

A glass substrate with ITO was washed, and a tin(II) oxide colloidal solution (15% water dispersion, manufactured by Alfa Aesar) diluted fivefold was applied thereon by spin coating, followed by heating at 150°C for 30 minutes to form an electron-transporting layer on a thin film having a thickness of 16 nm.

### [Formation of Photoelectric Conversion Layer]

0.487 Gram of lead bromide, 1.034 g of formamidinium iodide, 2.903 g of lead iodide, and 0.139 g of methylammonium bromide were dissolved in 4.25 g of N,N-dimethylformamide and 1.216 g of dimethyl sulfoxide, and were stirred for 1 hour (solution 1). Further, 0.100 g of cesium iodide was dissolved in 0.285 g of dimethyl sulfoxide, and the solution was stirred for 1 hour (solution 2). After that, the cesium iodide solution (solution 2) was added to the solution 1 to prepare a coating liquid for a photoelectric conversion layer. The coating liquid was applied onto the electron-transporting layer by spin coating to form a photoelectric conversion layer formed of Cs_{0.05}(FA_{0.83}MA_{0.17})_{0.95}Pb(I_{0.83}Br_{0.17})₃ and having a thickness of 600 nm.

### [Formation of Charge-transporting Layer]

0.1 Gram of the particle 1 and 0.01 g of a calixarene compound (Japanese Patent Laid-Open No. 2003-207913) were mixed with 10.6 g of 2-propanol, and 11 g of beads (zirconia beads, Torayceram (trademark) zirconia beads, 0.3 mm) were loaded into the mixture, followed by paint shaker dispersion (manufactured by Toyo Seiki Co., Ltd.) for 3 hours. After that, 0.2 g of the resin solution 1 was added thereto, and paint shaker dispersion was performed again for 4 hours to prepare a coating liquid for a charge-transporting layer. The coating liquid for a charge-transporting layer was spin-coated on the photoelectric conversion layer to form a charge-transporting layer having a thickness of 180 nm.

### [Formation of Second Charge-transporting Layer]

0.15 Gram of Spiro-OMeTAD serving as a material for a second charge-transporting layer was dissolved in 2.2 g of chlorobenzene. 36 Microliters of an acetonitrile solution obtained by dissolving 0.2 g of lithium-bis(trifluoromethanesulfonyl)imide in 0.3 g of acetonitrile and 60 µL of 4-tert-butylpyridine (TBP) were added to the chlorobenzene solution, and the contents were mixed. Further, 58 µL of an acetonitrile solution obtained by dissolving 0.11 g of [tris(2-(1H-pyrazol-1-yl)-4-tert-butylpyridine)cobalt(III) tris(bis(trifluoromethylsulfonyl)imide)] in 0.3 g of acetonitrile was mixed thereinto to prepare a coating liquid for a second charge-transporting layer. The coating liquid for a charge-transporting layer was applied onto the above-mentioned charge-transporting layer by a spin coating method to form a second charge-transporting layer having a thickness of 100 nm.

### [Formation of First Electrode]

A gold electrode having a thickness of 80 nm and an area of 0.09 cm² was formed on the second charge-transporting layer by a vacuum vapor deposition method. Thus, a photoelectric conversion element 1 was obtained. A small electrode was adopted as a first electrode in each of Examples according to the present invention.

### [Analysis of Amount of Compound]

The electrode surface of the photoelectric conversion element was peeled off to expose the surface of the charge-transporting layer. The surface of the charge-transporting layer was wiped with a cotton swab or the like with a solvent, dissolved in deuterated sulfuric acid, and subjected to ¹H-NMR measurement (apparatus: AVANCE3-500 manufactured by BRUKER). In addition, the mass and structure analysis of the peeled-off charge-transporting layer components was performed by elemental analysis, such as GPC and MALDI-TOF-MS, IR, gas chromatography, XPS, and EDX, to recognize the presence of a compound.

In addition, the thickness was observed with a cross-sectional SEM (apparatus: SmartSEM manufactured by Carl Zeiss Co., Ltd.) after the cutting of the photoelectric conversion element and the fixing of the sample to a tilted sample stage.

### [Analysis of Particle Size of Charge-transporting Material]

The particle size of a charge-transporting material is a number-average particle size in a particle size distribution. In the present invention, the particle size of the charge-transporting material was derived by an image imaging method using a TEM.

Specifically, first, N particles (N represents 1,000 or more) were extracted by using image processing software Photoshop (manufactured by Adobe Inc.) through use of a TEM image of the resultant cross section of the charge-transporting layer. Next, the area S of each particle was determined, the diameter of a circle with the same area as the area (=2×(S/π)^{1/2}) was adopted as the particle size, and an average value of the central 80% of the N particles was adopted.

The device configuration of the photoelectric conversion element 1 is shown in Table 2.

### (Production of Photoelectric Conversion Elements 2 to 7 and 21)

Photoelectric conversion elements 2 to 7 and 21 are each obtained in the same manner as in the photoelectric conversion element 1 except that the volume ratio of the charge-transporting material to the insulating resin is changed to a value shown in Table 2.

### (Production of Photoelectric Conversion Element 8)

A photoelectric conversion element 8 is obtained in the same manner as in the photoelectric conversion element 1 except that, in the formation of the charge-transporting layer, the coating liquid for a charge-transporting layer obtained by the paint shaker dispersion is further centrifuged (at 15,000 rpm for 6 minutes) so that the particle size in the dispersion may be reduced, followed by the adjustment of the volume ratio of the charge-transporting material to the insulating resin to 10 times.

### (Production of Photoelectric Conversion Element 9)

A photoelectric conversion element 9 is obtained in the same manner as in the photoelectric conversion element 1 except that the polyvinyl butyral (product name: BM-2, manufactured by Sekisui Chemical Co., Ltd.) is changed to polyvinyl butyral (product name: BX-1, manufactured by Sekisui Chemical Co., Ltd.).

### (Production of Photoelectric Conversion Element 10)

A photoelectric conversion element 10 is obtained in the same manner as in the photoelectric conversion element 1 except that the calixarene compound is changed to 2-naphthol.

### (Production of Photoelectric Conversion Element 11)

A photoelectric conversion element 11 is obtained in the same manner as in the photoelectric conversion element 1 except that the particle 1 is changed to a nickel phthalocyanine particle.

### (Production of Photoelectric Conversion Element 12)

A photoelectric conversion element 12 is obtained in the same manner as in the photoelectric conversion element 1 except that the second charge-transporting layer is not arranged.

### (Production of Photoelectric Conversion Element 13)

A photoelectric conversion element 13 is obtained in the same manner as in the photoelectric conversion element 1 except that the calixarene compound is not used.

### (Production of Photoelectric Conversion Element 14)

A photoelectric conversion element 14 is obtained in the same manner as in the photoelectric conversion element 1 except that the polyvinyl butyral is changed to polymethyl methacrylate (PMMA, manufactured by Sigma-Aldrich Co. LLC, glass transition temperature: 70°C).

### (Production of Photoelectric Conversion Element 15)

A photoelectric conversion element 15 is obtained in the same manner as in the photoelectric conversion element 1 except that the polyvinyl butyral is changed to polymethyl methacrylate (PMMA, manufactured by Sigma-Aldrich Co. LLC, glass transition temperature: 100°C).

### (Production of Photoelectric Conversion Element 16)

A photoelectric conversion element 16 is obtained in the same manner as in the photoelectric conversion element 1 except that the particle 1 is changed to a particle containing a compound represented by the following formula (Pc-3).

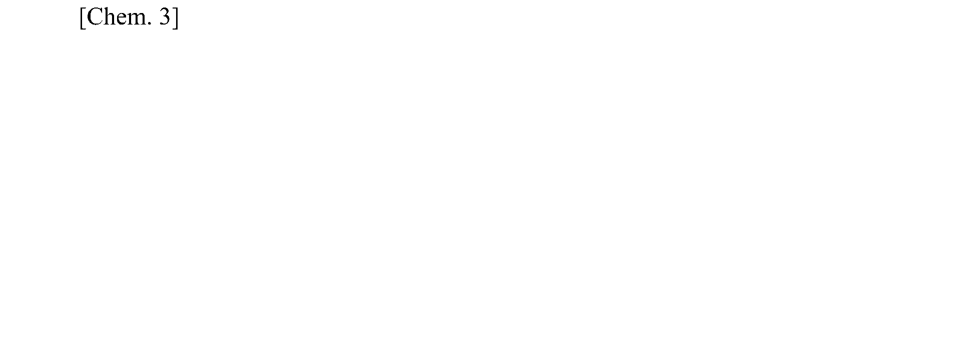

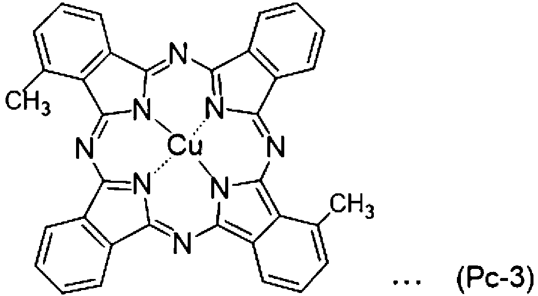

### (Production of Photoelectric Conversion Element 17)

A photoelectric conversion element 17 is obtained in the same manner as in the photoelectric conversion element 1 except that the particle 1 is changed to a quinacridone particle.

### (Production of Photoelectric Conversion Element 18)

A photoelectric conversion element 18 is obtained in the same manner as in Example 1 except that the composition of the photoelectric conversion layer is changed to MAPbI₃.

### (Production of Photoelectric Conversion Element 19)

A photoelectric conversion element 19 is obtained in the same manner as in the photoelectric conversion element 1 except that: a layer similar to the second charge-transporting layer formed in the photoelectric conversion element 1 is formed as the charge-transporting layer of the photoelectric conversion element 19; and the second charge-transporting layer is not arranged on the charge-transporting layer.

### (Production of Photoelectric Conversion Element 20)

A photoelectric conversion element 20 is obtained in the same manner as in the photoelectric conversion element 1 except that the particle 1 is not used.

### (Production of Photoelectric Conversion Element 22)

A photoelectric conversion element 22 is obtained in the same manner as in the photoelectric conversion element 15 except that: the particle 1 is changed to Spiro-OMeTAD; the volume ratio of the charge-transporting material to the insulating resin is set to 1; the thickness of the charge-transporting layer is set to 100 nm; the calixarene compound is not used; and the second charge-transporting layer is not arranged on the charge-transporting layer.

### (Production of Photoelectric Conversion Element 23)

A photoelectric conversion element 23 is obtained in the same manner as in the photoelectric conversion element 22 except that the thickness of the charge-transporting layer is set to 180 nm.

### (Production of Photoelectric Conversion Element 24)

A photoelectric conversion element 24 is obtained in the same manner as in the photoelectric conversion element 22 except that the thickness of the charge-transporting layer is set to 300 nm.

### (Production of Photoelectric Conversion Element 25)

A photoelectric conversion element 25 is obtained in the same manner as in the photoelectric conversion element 15 except that: the particle 1 is changed to Spiro-OMeTAD; the thickness of the charge-transporting layer is set to 180 nm; and the second charge-transporting layer is not arranged on the charge-transporting layer.

### (Production of Photoelectric Conversion Element 26)

A photoelectric conversion element 26 is obtained in the same manner as in the photoelectric conversion element 1 except that: the particle 1 is changed to a nickel(II) phthalocyanine-tetrasulfonic acid tetrasodium salt particle; and the insulating resin is changed to PEDOT:PSS.

### (Production of Photoelectric Conversion Element 27)

A photoelectric conversion element 27 is obtained in the same manner as in the photoelectric conversion element 1 except that: the particle 1 is changed to a particle containing a compound represented by the following formula (Pc-4); and the insulating resin is changed to P3HT.

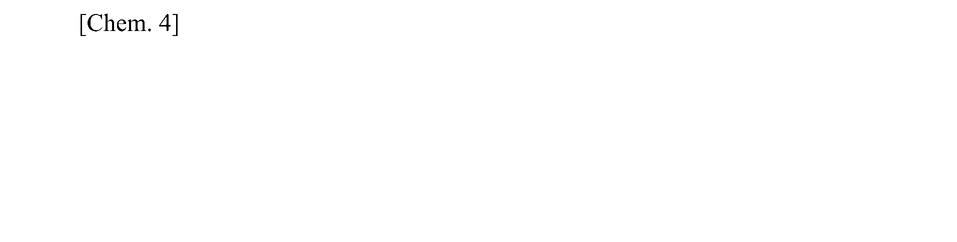

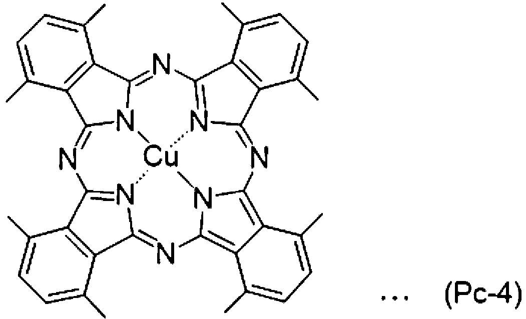

### (Example 1)

### [Evaluation]

The photoelectric conversion element 1 produced in the section (Production of Photoelectric Conversion Element) was subjected to impedance measurement. ModuLab XM MTS (manufactured by Solartron Analytical) was used for the measurement. The resultant impedance data was fitted by using the equivalent circuit illustrated in Fig. 4 as shown in Fig. 2 as an example (Fig. 2 is an example of the photoelectric conversion element 4), and the values of R_{rec}×S [Ω·cm²], R_{rec}/R_{ct}, P_{rec}, and P_{ct} were determined. The results are shown in Table 3.

Next, a power supply (236 model, manufactured by Keithley Instruments) was connected between the electrodes of the photoelectric conversion element 1, and its initial photoelectric conversion efficiency was measured by: irradiating the element with constant light through use of a solar simulator (manufactured by Yamashita Denso Corporation) having an intensity of 110 mW/cm²; and measuring the generated current and voltage. In addition, each of ten electrodes for each element was subjected to the measurement, and the average of the measured values was adopted as the representative value of the element. The results are shown in Table 3. After that, light of 10,000 Lx was continuously applied to the element with a white LED, and its photoelectric conversion efficiency after 50 days was measured. Then, the maintenance rate of the photoelectric conversion efficiency after 50 days with respect to the resultant initial photoelectric conversion efficiency was evaluated. The results are shown in Table 3.

In Table 3, the photoelectric conversion efficiency in Example 1 was set to 100%, and a ratio thereto was shown as the conversion efficiency of each element.

The photoelectric conversion elements of Examples 2 to 18 and Comparative Examples 1 to 9 are each evaluated for its initial photoelectric conversion efficiency and its maintenance rate of the photoelectric conversion efficiency in the same manner as in Example 1. The results are shown in Table 3.

In Comparative Example 2, the initial photoelectric conversion efficiency was not measured, and hence the maintenance rate of photoelectric conversion was not was able to be evaluated.

### [Table 2]

**Table 2**

| Photoelectric conversion element No. | Photoelectric conversion layer | Charge-transporting material | | Resin | | Volume ratio of charge-transporting material to resin | Aromatic ring compound having hydroxy group | Second charge-transporting layer |
|---|---|---|---|---|---|---|---|---|
| | Compound | Compound | Particle size /nm | Compound | Glass transition temperature /°C | | | |
| 1 | Cs_{0.05}(FA_{0.83}MA_{0.17})_{0.95}Pb(I_{0.83}Br_{0.17})₃ | HOGaPc | 2.3×10² | BM-2 | 71 | 10 | Calixarene compound | Present |
| 2 | Cs_{0.05}(FA_{0.83}MA_{0.17})_{0.95}Pb(I_{0.83}Br_{0.17})₃ | HOGaPc | 1.2×10² | BM-2 | 71 | 3 | Calixarene compound | Present |
| 3 | Cs_{0.05}(FA_{0.83}MA_{0.17})_{0.95}Pb(I_{0.83}Br_{0.17})₃ | HOGaPc | 1.4×10² | BM-2 | 71 | 5 | Calixarene compound | Present |
| 4 | Cs_{0.05}(FA_{0.83}MA_{0.17})_{0.95}Pb(I_{0.83}Br_{0.17})₃ | HOGaPc | 1.9×10² | BM-2 | 71 | 8 | Calixarene compound | Present |
| 5 | Cs_{0.05}(FA_{0.83}MA_{0.17})_{0.95}Pb(I_{0.83}Br_{0.17})₃ | HOGaPc | 2.6×10² | BM-2 | 71 | 13 | Calixarene compound | Present |
| 6 | Cs_{0.05}(FA_{0.83}MA_{0.17})_{0.95}Pb(I_{0.83}Br_{0.17})₃ | HOGaPc | 2.8×10² | BM-2 | 71 | 15 | Calixarene compound | Present |
| 7 | Cs_{0.05}(FA_{0.83}MA_{0.17})_{0.95}Pb(I_{0.83}Br_{0.17})₃ | HOGaPc | 3.7×10² | BM-2 | 71 | 20 | Calixarene compound | Present |
| 8 | Cs_{0.05}(FA_{0.83}MA_{0.17})_{0.95}Pb(I_{0.83}Br_{0.17})₃ | HOGaPc | 1.5×10¹ | BM-2 | 71 | 10 | Calixarene compound | Present |
| 9 | Cs_{0.05}(FA_{0.83}MA_{0.17})_{0.95}Pb(I_{0.83}Br_{0.17})₃ | HOGaPc | 2.1×10² | BX-1 | 95 | 10 | Calixarene compound | Present |
| 10 | Cs_{0.05}(FA_{0.83}MA_{0.17})_{0.95}Pb(I_{0.83}Br_{0.17})₃ | HOGaPc | 3.0×10² | BM-2 | 71 | 10 | 2-naphthol | Present |
| 11 | Cs_{0.05}(FA_{0.83}MA_{0.17})_{0.95}Pb(I_{0.83}Br_{0.17})₃ | NiPc | 3.3×10² | BM-2 | 71 | 10 | Calixarene compound | Present |
| 12 | Cs_{0.05}(FA_{0.83}MA_{0.17})_{0.95}Pb(I_{0.83}Br_{0.17})₃ | HOGaPc | 2.3×10² | BM-2 | 71 | 10 | Calixarene compound | Absent |
| 13 | Cs_{0.05}(FA_{0.83}MA_{0.17})_{0.95}Pb(I_{0.83}Br_{0.17})₃ | HOGaPc | 3.6×10² | BM-2 | 71 | 10 | - | Present |
| 14 | Cs_{0.05}(FA_{0.83}MA_{0.17})_{0.95}Pb(I_{0.83}Br_{0.17})₃ | HOGaPc | 2.9×10² | PMMA | 70 | 10 | Calixarene compound | Present |
| 15 | Cs_{0.05}(FA_{0.83}MA_{0.17})_{0.95}Pb(I_{0.83}Br_{0.17})₃ | HOGaPc | 3.0×10² | PMMA | 100 | 10 | Calixarene compound | Present |
| 16 | Cs_{0.05}(FA_{0.83}MA_{0.17})_{0.95}Pb(I_{0.83}Br_{0.17})₃ | Pc-3 | 3.7×10¹ | BM-2 | 71 | 10 | Calixarene compound | Present |
| 17 | Cs_{0.05}(FA_{0.83}MA_{0.17})_{0.95}Pb(I_{0.83}Br_{0.17})₃ | Quinacridone | 1.3×10² | BM-2 | 71 | 10 | Calixarene compound | Present |
| 18 | MAPbI₃ | HOGaPc | 2.3×10² | BM-2 | 71 | 10 | Calixarene compound | Present |
| 19 | Cs_{0.05}(FA_{0.83}MA_{0.17})_{0.95}Pb(I_{0.83}Br_{0.17})₃ | Spiro | - | - | - | - | - | Absent |
| 20 | Cs_{0.05}(FA_{0.83}MA_{0.17})_{0.95}Pb(I_{0.83}Br_{0.17})₃ | - | - | BM-2 | 71 | 0 | - | Present |
| 21 | Cs_{0.05}(FA_{0.83}MA_{0.17})_{0.95}Pb(I_{0.83}Br_{0.17})₃ | HOGaPc | 1.1×10² | BM-2 | 71 | 1 | Calixarene compound | Present |
| 22 | Cs_{0.05}(FA_{0.83}MA_{0.17})_{0.95}Pb(I_{0.83}Br_{0.17})₃ | Spiro | - | PMMA | 100 | 1 | - | Absent |
| 23 | Cs_{0.05}(FA_{0.83}MA_{0.17})_{0.95}Pb(I_{0.83}Br_{0.17})₃ | Spiro | - | PMMA | 100 | 1 | - | Absent |
| 24 | Cs_{0.05}(FA_{0.83}MA_{0.17})_{0.95}Pb(I_{0.83}Br_{0.17})₃ | Spiro | - | PMMA | 100 | 1 | - | Absent |
| 25 | Cs_{0.05}(FA_{0.83}MA_{0.17})_{0.95}Pb(I_{0.83}Br_{0.17})₃ | Spiro | - | PMMA | 100 | 10 | Calixarene compound | Absent |
| 26 | Cs_{0.05}(FA_{0.83}MA_{0.17})_{0.95}Pb(I_{0.83}Br_{0.17})₃ | Nickel(II) phthalocyanine-tetrasulfonic acid tetrasodium salt | - | PEDOT:PSS | - | 10 | Calixarene compound | Present |
| 27 | Cs_{0.05}(FA_{0.83}MA_{0.17})_{0.95}Pb(I_{0.83}Br_{0.17})₃ | Pc-4 | - | P3HT | - | 10 | Calixarene compound | Present |

### [Table 3]

**Table 3**

| No. | Photoelectric conversion element No. | Maximum values of phase in low frequency and high frequency ranges | *R*_{rec}×*S* /Ω·cm² | *R*_{rec}/*R*_{ct} | *P*_{rec} | *P*_{ct} | Initial photoelectric conversion efficiency /% | Maintenance rate of photoelectric conversion efficiency /% |
|---|---|---|---|---|---|---|---|---|
| Example 1 | 1 | Present | 8.5×10⁵ | 85 | 0.83 | 0.91 | 100 | 97.8 |
| Example 2 | 2 | Present | 1.1×10⁶ | 39 | 0.78 | 0.87 | 84 | 85.7 |
| Example 3 | 3 | Present | 6.1×10⁵ | 42 | 0.81 | 0.91 | 89 | 96.3 |
| Example 4 | 4 | Present | 5.5×10⁵ | 34 | 0.81 | 0.94 | 101 | 96.8 |
| Example 5 | 5 | Present | 6.7×10⁵ | 57 | 0.81 | 0.96 | 95 | 97.1 |
| Example 6 | 6 | Present | 5.4×10⁵ | 37 | 0.80 | 0.98 | 92 | 95.7 |
| Example 7 | 7 | Present | 4.5×10⁵ | 46 | 0.80 | 0.94 | 100 | 96.9 |
| Example 8 | 8 | Present | 9.8×10⁵ | 92 | 0.90 | 0.95 | 97 | 97.4 |
| Example 9 | 9 | Present | 7.3×10⁵ | 60 | 0.82 | 0.89 | 98 | 97.6 |
| Example 10 | 10 | Present | 8.1×10⁵ | 79 | 0.84 | 0.92 | 98 | 97.6 |
| Example 11 | 11 | Present | 6.8×10⁵ | 51 | 0.83 | 0.95 | 97 | 97.5 |
| Example 12 | 12 | Present | 8.9×10⁴ | 27 | 0.81 | 0.94 | 81 | 88.2 |
| Example 13 | 13 | Present | 3.8×10⁵ | 27 | 0.79 | 0.90 | 94 | 89.0 |
| Example 14 | 14 | Present | 2.6×10⁶ | 33 | 0.84 | 0.97 | 82 | 90.1 |
| Example 15 | 15 | Present | 1.7×10⁶ | 31 | 0.77 | 0.97 | 81 | 85.7 |
| Example 16 | 16 | Present | 6.8×10⁵ | 51 | 0.83 | 0.95 | 93 | 89.9 |
| Example 17 | 17 | Present | 1.3×10⁶ | 48 | 0.79 | 0.93 | 93 | 85.3 |
| Example 18 | 18 | Present | 8.2×10⁵ | 82 | 0.82 | 0.91 | 97 | 97.5 |
| Comparative Example 1 | 19 | Present | 3.6×10⁵ | 20 | 0.83 | 0.96 | 76 | 62.6 |
| Comparative Example 2 | 20 | Present | 5.9×10⁶ | 12 | 0.83 | 0.95 | 0 | - |
| Comparative Example 3 | 21 | Present | 1.5×10⁶ | 20 | 0.74 | 0.82 | 18 | 71.1 |
| Comparative Example 4 | 22 | Absent | 1.8×10³ | - | 0.71 | - | 78 | 74.2 |
| Comparative Example 5 | 23 | Present | 2.5×10⁴ | 2 | 0.79 | 0.94 | 76 | 76.7 |
| Comparative Example 6 | 24 | Present | 2.4×10⁵ | 11 | 0.80 | 0.86 | 72 | 78.1 |
| Comparative Example 7 | 25 | Present | 1.1×10³ | 1 | 0.88 | 0.98 | 78 | 71.3 |
| Comparative Example 8 | 26 | Absent | 4.4×10³ | - | 0.79 | - | 77 | 72.2 |
| Comparative Example 9 | 27 | Present | 6.5×10³ | 13 | 0.78 | 0.89 | 75 | 75.3 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, and Japanese Patent Application No. 2024-085998 filed on May 28, 2024, and the entire contents thereof are incorporated herein by reference.

### [Reference Signs List]

1 photoelectric conversion element
2 substrate
3 second electrode
4 electron-transporting layer
5 photoelectric conversion layer
6 charge-transporting layer
7 first electrode
30 moving body
31, 41 photoelectric conversion element
32 body
40 building material
42 protective member
43 heat dissipation member
44a, 44b exterior

## Claims

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein, when a smaller one of an electrode area of the first electrode and an electrode area of the second electrode is represented by S [cm²], and an impedance is measured by applying an AC voltage with a DC voltage component V_{DC}=0 [V] and a root-mean-square of an AC voltage component Vᵣₘₛ=50 [mV] to the photoelectric conversion element while changing a frequency thereof from 1.0×10⁻² [Hz] to 1.0×10⁶ [Hz], in a plot in which a horizontal axis represents a frequency [Hz] and a vertical axis represents a phase [deg] based on measurement results of the impedance, a phase of the impedance has a maximum value in a low frequency range of 1.0×10⁻² to less than 1.0×10² [Hz], and the phase of the impedance has a maximum value in a high frequency range of 1.0×10² to 1.0×10⁶ [Hz], and
wherein, in a Nyquist plot in which a horizontal axis represents an impedance real part Z' [Ω] and a vertical axis represents an impedance imaginary part Z" [Ω] based on the measurement results of the impedance, a maximum value R_{rec} [Ω] out of resistance values obtained by fitting an arc corresponding to the maximum value of the phase in the low frequency range with a parallel circuit of a resistance element and a constant phase element satisfies the following formula (E1): $1.0 \times {10}^{4} \leq {R}_{rec} \times S \leq 1.0 \times {10}^{7}$
and a maximum value R_{ct} [Ω] out of resistance values obtained by fitting an arc corresponding to the maximum value of the phase in the high frequency range with the parallel circuit of the resistance element and the constant phase element, and the R_{rec} [Ω] satisfy the following formula (E2). ${R}_{rec} / {R}_{ct} \geq 25$

2. The photoelectric conversion element according to claim 1, wherein an exponent P_{rec} of the constant phase element obtained by the fitting performed to obtain the R_{rec} [Ω] satisfies the following formula (E3). ${P}_{rec} \geq 0.80$

3. The photoelectric conversion element according to claim 1 or 2, wherein an exponent P_{ct} of the constant phase element obtained by the fitting performed to obtain the R_{ct} [Ω] satisfies the following formula (E4). ${P}_{ct} \leq 0.95$

4. The photoelectric conversion element according to any one of claims 1 to 3, wherein the R_{rec} [Ω] satisfies the following formula (E5). $1.0 \times {10}^{5} < {R}_{rec} \times S < 1.0 \times {10}^{6}$

5. The photoelectric conversion element according to any one of claims 1 to 4, wherein the R_{ct} [Ω] and the R_{rec} [Ω] satisfy the following formula (E6). ${R}_{rec} / {R}_{ct} \geq 50$

6. The photoelectric conversion element according to any one of claims 1 to 5,
wherein the photoelectric conversion element further comprises a charge-transporting layer between the photoelectric conversion layer and the first electrode, and
wherein the charge-transporting layer contains a charge-transporting material and an insulating resin on a surface of the photoelectric conversion layer.

7. The photoelectric conversion element according to claim 6, wherein the charge-transporting material is a charge-transporting particle.

8. The photoelectric conversion element according to claim 7, wherein the charge-transporting particle has an average particle size of 10 to 500 nm.

9. The photoelectric conversion element according to claim 6, wherein a volume ratio of the charge-transporting material to the insulating resin is 5 to 30 times.

10. The photoelectric conversion element according to claim 6, wherein the charge-transporting material is a phthalocyanine compound.

11. The photoelectric conversion element according to claim 10, wherein the phthalocyanine compound has a structure represented by the following formula (Pc-2): 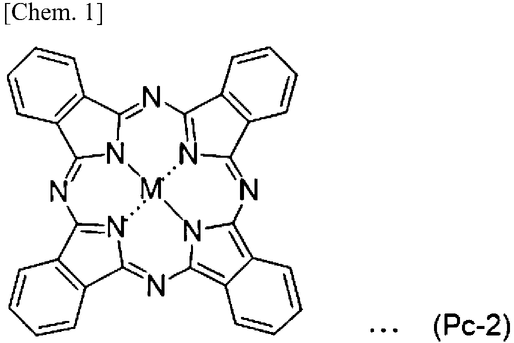 wherein M in the formula (Pc-2) represents H₂ or a metal atom that may have a ligand.

12. The photoelectric conversion element according to claim 6, wherein the insulating resin has a glass transition temperature of 95°C or less.

13. The photoelectric conversion element according to claim 6, wherein the insulating resin is a polyvinyl acetal resin or a polyvinyl butyral resin.

14. The photoelectric conversion element according to claim 6, wherein the charge-transporting layer contains an aromatic ring compound having a hydroxy group, the aromatic ring compound being different from the charge-transporting material and the insulating resin.

15. The photoelectric conversion element according to claim 6, wherein the photoelectric conversion element further comprises a second charge-transporting layer between the first electrode and the charge-transporting layer.

16. A photoelectric conversion apparatus comprising the photoelectric conversion element of any one of claims 1 to 15.

## Amended claims

### Amended claims under Art. 19.1 PCT

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein, when a smaller one of an electrode area of the first electrode and an electrode area of the second electrode is represented by S [cm²], and an impedance is measured by applying an AC voltage with a DC voltage component V_{DC}=0 [V] and a root-mean-square of an AC voltage component Vᵣₘₛ=50 [mV] to the photoelectric conversion element while changing a frequency thereof from 1.0×10⁻² [Hz] to 1.0×10⁶ [Hz], in a plot in which a horizontal axis represents a frequency [Hz] and a vertical axis represents a phase [deg] based on measurement results of the impedance, a phase of the impedance has a maximum value in a low frequency range of 1.0×10⁻² to less than 1.0×10² [Hz], and the phase of the impedance has a maximum value in a high frequency range of 1.0×10² to 1.0×10⁶ [Hz], and
wherein, in a Nyquist plot in which a horizontal axis represents an impedance real part Z' [Ω] and a vertical axis represents an impedance imaginary part Z" [Ω] based on the measurement results of the impedance, a maximum value R_{rec} [Ω] out of resistance values obtained by fitting an arc corresponding to the maximum value of the phase in the low frequency range with a parallel circuit of a resistance element and a constant phase element satisfies the following formula (E1): $1.0 \times {10}^{4} \leq {R}_{rec} \times S \leq 1.0 \times {10}^{7}$
, a maximum value R_{ct} [Ω] out of resistance values obtained by fitting an arc corresponding to the maximum value of the phase in the high frequency range with the parallel circuit of the resistance element and the constant phase element, and the R_{rec} [Ω] satisfy the following formula (E2): ${R}_{rec} / {R}_{ct} \geq 25$
and an exponent P_{rec} of the constant phase element obtained by the fitting performed to obtain the R_{rec} [Ω] satisfies the following formula (E3). ${P}_{rec} \geq 0.80$

2. The photoelectric conversion element according to claim 1, wherein an exponent P_{ct} of the constant phase element obtained by the fitting performed to obtain the R_{ct} [Ω] satisfies the following formula (E4). ${P}_{ct} \leq 0.95$

3. The photoelectric conversion element according to claim 1 or 3, wherein the R_{rec} [Ω] satisfies the following formula (E5). $1.0 \times {10}^{5} < {R}_{rec} \times S < 1.0 \times {10}^{6}$

4. The photoelectric conversion element according to any one of claims 1, 3 and 4, wherein the R_{ct} [Ω] and the R_{rec} [Ω] satisfy the following formula (E6). ${R}_{rec} / {R}_{ct} \geq 50$

5. The photoelectric conversion element according to any one of claims 1 and 3 to 5,
wherein the photoelectric conversion element further comprises a charge-transporting layer between the photoelectric conversion layer and the first electrode, and
wherein the charge-transporting layer contains a charge-transporting material and an insulating resin on a surface of the photoelectric conversion layer.

6. The photoelectric conversion element according to claim 6, wherein the charge-transporting material is a charge-transporting particle.

7. The photoelectric conversion element according to claim 7, wherein the charge-transporting particle has an average particle size of 10 to 500 nm.

8. The photoelectric conversion element according to any one of claims 6 to 8, wherein a volume ratio of the charge-transporting material to the insulating resin is 5 to 30 times.

9. The photoelectric conversion element according to any one of claims 6 to 9, wherein the charge-transporting material is a phthalocyanine compound.

10. The photoelectric conversion element according to claim 10, wherein the phthalocyanine compound has a structure represented by the following formula (Pc-2): 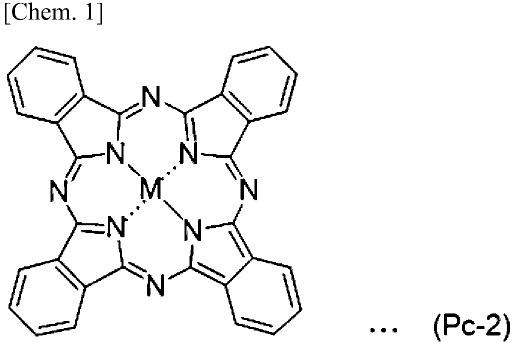 wherein M in the formula (Pc-2) represents H₂ or a metal atom that may have a ligand.

11. The photoelectric conversion element according to any one of claims 6 to 11, wherein the insulating resin has a glass transition temperature of 95°C or less.

12. The photoelectric conversion element according to any one of claims 6 to 12, wherein the insulating resin is a polyvinyl acetal resin or a polyvinyl butyral resin.

13. The photoelectric conversion element according to any one of claims 6 to 13, wherein the charge-transporting layer contains an aromatic ring compound having a hydroxy group, the aromatic ring compound being different from the charge-transporting material and the insulating resin.

14. The photoelectric conversion element according to any one of claims 6 to 14, wherein the photoelectric conversion element further comprises a second charge-transporting layer between the first electrode and the charge-transporting layer.

15. A photoelectric conversion apparatus comprising the photoelectric conversion element of any one of claims 1 and 3 to 15.

16. A photoelectric conversion element comprising:
a first electrode;
a second electrode; and
a photoelectric conversion layer arranged between the first electrode and the second electrode, the photoelectric conversion layer containing a crystal having a perovskite structure,
wherein, the photoelectric conversion element further comprises a charge-transporting layer between the photoelectric conversion layer and the first electrode,
wherein the charge-transporting layer contains a charge-transporting material and an insulating resin on a surface of the photoelectric conversion layer,
wherein, when a smaller one of an electrode area of the first electrode and an electrode area of the second electrode is represented by S [cm²], and an impedance is measured by applying an AC voltage with a DC voltage component V_{DC}=0 [V] and a root-mean-square of an AC voltage component Vᵣₘₛ=50 [mV] to the photoelectric conversion element while changing a frequency thereof from 1.0×10⁻² [Hz] to 1.0×10⁶ [Hz], in a plot in which a horizontal axis represents a frequency [Hz] and a vertical axis represents a phase [deg] based on measurement results of the impedance, a phase of the impedance has a maximum value in a low frequency range of 1.0×10⁻² to less than 1.0×10² [Hz], and the phase of the impedance has a maximum value in a high frequency range of 1.0×10² to 1.0×10⁶ [Hz], and
wherein, in a Nyquist plot in which a horizontal axis represents an impedance real part Z' [Ω] and a vertical axis represents an impedance imaginary part Z" [Ω] based on the measurement results of the impedance, a maximum value R_{rec} [Ω] out of resistance values obtained by fitting an arc corresponding to the maximum value of the phase in the low frequency range with a parallel circuit of a resistance element and a constant phase element satisfies the following formula (E1): $1.0 \times {10}^{4} \leq {R}_{rec} \times S \leq 1.0 \times {10}^{7}$
and a maximum value R_{ct} [Ω] out of resistance values obtained by fitting an arc corresponding to the maximum value of the phase in the high frequency range with the parallel circuit of the resistance element and the constant phase element, and the R_{rec} [Ω] satisfy the following formula (E2). ${R}_{rec} / {R}_{ct} \geq 25$

17. The photoelectric conversion element according to claim 17, wherein an exponent P_{ct} of the constant phase element obtained by the fitting performed to obtain the R_{ct} [Ω] satisfies the following formula (E4). ${P}_{ct} \leq 0.95$

18. The photoelectric conversion element according to any one of claim 17, wherein the R_{rec} [Ω] satisfies the following formula (E5). $1.0 \times {10}^{5} < {R}_{rec} \times S < 1.0 \times {10}^{6}$

19. The photoelectric conversion element according to any one of claim 17, wherein the R_{ct} [Ω] and the R_{rec} [Ω] satisfy the following formula (E6). ${R}_{rec} / {R}_{ct} \geq 50$

20. The photoelectric conversion element according to claim17, wherein the charge-transporting material is a charge-transporting particle.

21. The photoelectric conversion element according to claim 17, wherein the charge-transporting particle has an average particle size of 10 to 500 nm.

22. The photoelectric conversion element according to claim17, wherein a volume ratio of the charge-transporting material to the insulating resin is 5 to 30 times.

23. The photoelectric conversion element according to claim17, wherein the charge-transporting material is a phthalocyanine compound.

24. The photoelectric conversion element according to claim24, wherein the phthalocyanine compound has a structure represented by the following formula (Pc-2): 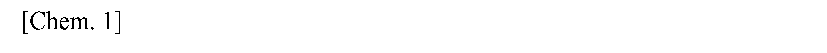 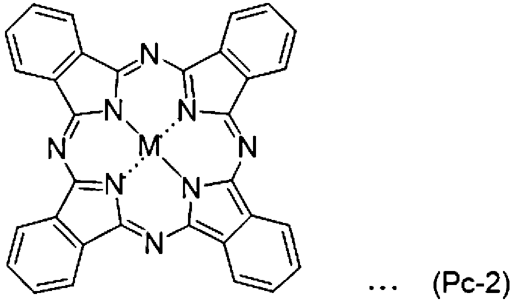 wherein M in the formula (Pc-2) represents H₂ or a metal atom that may have a ligand.

25. The photoelectric conversion element according to claim17, wherein the insulating resin has a glass transition temperature of 95°C or less.

26. The photoelectric conversion element according to claim 17, wherein the insulating resin is a polyvinyl acetal resin or a polyvinyl butyral resin.

27. The photoelectric conversion element according to claim17, wherein the charge-transporting layer contains an aromatic ring compound having a hydroxy group, the aromatic ring compound being different from the charge-transporting material and the insulating resin.

28. The photoelectric conversion element according to claim 17, wherein the photoelectric conversion element further comprises a second charge-transporting layer between the first electrode and the charge-transporting layer.

29. A photoelectric conversion apparatus comprising the photoelectric conversion element of any one of claims 17 to 29.

Statement under Art. 19.1 PCT
Original claims 1 to 16 have been replaced with new claims.

Amended claim 1 is based on original claims 1 and 2.

Amended claims 3 to 6, 9, 10, and 12 to 16 have been amended to change their dependency relationships due to the deletion of claim 2.

New claim 17 is based on original claims 1 and 6.

New claims 18 to 30 are based on original claims 3 to 5 and 7 to16.
